# EUROPEAN PATENT SPECIFICATION

(11) **EP 3 419 505 B1**
(45) Date of publication and mention of the grant of the patent: **12.05.2021**
(21) Application number: 17712234.8
(22) Date of filing: 23.02.2017
(51) Int. Cl.: A61B 5/00, A61B 5/055, A61B 5/0205, A61B 5/021, A61B 5/0402, A61B 5/145, A61B 5/1455, G16H 10/60, G16H 40/67

(54) **SYSTEMS AND METHODS FOR RETRANSMITTING WIRELESS DATA STREAMS**
SYSTEME UND VERFAHREN ZUR NEUÜBERTRAGUNG VON DRAHTLOSDATENSTRÖMEN
SYSTÈMES ET PROCÉDÉS DE RETRANSMISSION DE FLUX DE DONNÉES SANS FIL

(30) Priority: 23.02.2016 US 201662298641 P
(43) Date of publication of application: 02.01.2019
(73) Proprietor: Koninklijke Philips N.V., 5656 AG Eindhoven (NL)
(72) Inventor: HAWKES, Calvert Tazewell, 5656 AE Eindhoven (NL); GEMMATI, Michael Angelo, Jr, 5656 AE Eindhoven (NL); THIJSSEN, Carolus Gerardus, 5656 AE Eindhoven (NL)
(74) Representative: de Haan, Poul Erik
(86) International application number: PCT/IB2017/051023
(87) International publication number: WO 2017/145077

(56) References cited:
- US-A1- 2003 058 502
- US-A1- 2012 016 612
- US-A1- 2012 215 092
- US-A1- 2014 275 970

## Description

### FIELD

The following relates to the medical imaging arts, magnetic resonance imaging arts, data transmission arts, and related arts.

### BACKGROUND

Wireless data is transmitted over radio frequencies in a hospital environment by sensors, providing remote monitoring of physiological parameters such as blood oxygen saturation (SpO2), electrocardiograms, temperature, blood pressure, and the like. For example, such wireless vital sign sensors are increasingly being used to monitor patients undergoing magnetic resonance imaging (MRI) procedures. Wireless sensors have a particular advantage in the MRI setting since a wired sensor connected with the patient in the MRI bore can pick up time-varying magnetic fields from radio frequency (RF) transmissions and/or magnetic field gradients applied during the MRI imaging. Such signal pickups can introduce noise and in severe instances can lead to heating and potential consequent patient injury.

During an MRI scan the sensors are connected to the patient. A monitor located in the magnet room can display the patient information. Some typical implementations employ a 2.4 GHz radio with relatively short range (i.e., 50-100 feet) to transmit vital sign data off the sensor unit. The wireless sensor transmissions are received by the radio of a patient monitor located inside the magnet room and displayed. However, in practice the radiological technician or other medical professional conducting the MRI examination may not be present in the magnet room during the imaging, but rather may be located in an adjacent MRI control room. The MRI machine is controlled from the control room, so the monitor is the control room for convenience. In addition, noise produced from an MRI machine during scanning can be noisy, so the technician prefers to keep the monitor in the quieter control room. It would be useful to be able to wheel the patient monitor from the magnet room into the control room to enable the technician to continue to view patient vital signs in real time, in the control room, during the MRI procedure. However, the magnet room is enclosed in a room-sized Faraday shield to limit detrimental emissions from the MRI apparatus and to limit outside RF interference from adversely impacting the MRI imaging. RF shielding of the magnet room interferes with, or entirely prevents, the wireless sensor transmissions from being received by the patient monitor in the control room.

US 2014/0275970 describes an MRI communication system. In an embodiment the system comprises a first interface module and a second interface module, which communicate with one another via a network cable. Sensors can communicate with the first interface module, and both modules can receive and transmit wireless data.

US 2003/0058502 describes a communication system comprising a first antenna and a second antenna that are connected by an antenna coupling. The communication system provides a communication link across a barrier to radio frequencies, e.g. in an MRI environment.

A related problem is that it may be desirable to send patient data to a Hospital Information System (HIS). The shielding of the magnet room makes this difficult.

The following provides new and improved apparatuses and methods which overcome the foregoing problems and others.

### BRIEF SUMMARY

The invention is defined by the independent claims. The dependent claims define advantageous embodiments.

In accordance with one aspect, an apparatus for processing physiological data streams broadcast by at least one associated physiological sensor is provided. The apparatus includes: a first hub including a first radio configured to receive an individual medical information data stream broadcast by the at least one associated sensor at a data stream carrier frequency and to transmit a non-electrical signal carrying the individual medical information data stream; a second hub including a second radio; and a non-electrical communication link between the first hub and the second hub via which the non-electrical signal carrying the individual medical information data stream is communicated from the first radio to the second radio. The second radio is configured to receive the non-electrical signal carrying the medical information data stream from the first radio and re-broadcast the medical information data stream at the data stream carrier frequency with a defined time lag respective to the broadcast by the at least one associated sensor, such that the individual medical information data stream broadcast by the at least one sensor and the re-broadcast can be distinguished, wherein the non-electrical communication link is a fiber optic cable.

In accordance with another aspect, a method for processing physiological data streams broadcast by at least one associated physiological sensor is provided. The method includes: receiving, with a first radio in the first hub, an individual physiological data stream broadcast by the at least one associated physiological sensor at a data stream carrier frequency; transmitting, with the first radio, a non-electrical signal carrying the individual physiological data stream to a second radio via a non-electrical communication link between the first radio and the second radio; receiving, with the second hub, the non-electrical signal carrying the physiological data stream from the first radio; and re-broadcasting, with the second radio, the physiological data stream at the data stream carrier frequency with a defined time lag respective to the broadcast by the at least one associated physiological sensor, such that the individual medical information data stream broadcast by the at least one sensor and the re-broadcast can be distinguished, wherein the non-electrical signal is transmitted via a fiber optic cable connecting the first hub and the second hub.

One advantage resides in uninhibited transmission of a broadcasted signal and a re-broadcasted signal between multiple rooms.

Another advantage resides in moving a patient monitor between multiple rooms without interrupting transmission of data to the patient monitor.

Another advantage resides in combining multiple data streams into a single data stream and transmitting the single data stream between multiple rooms.

Further advantages of the present invention will be appreciated to those of ordinary skill in the art upon reading and understand the following detailed description. It will be appreciated that any given embodiment may achieve none, one, more, or all of the foregoing advantages and/or may achieve other advantages.

### BRIEF DESCRIPTION OF THE DRAWINGS

The invention may take form in various components and arrangements of components, and in various steps and arrangements of steps. The drawings are only for purposes of illustrating the preferred embodiments and are not to be construed as limiting the invention which is defined by the appended claims.
FIGURE 1 shows an embodiment of an apparatus to retransmit data streams in accordance with one aspect of the present disclosure.
FIGURE 2 shows an example of an operation of the apparatus of FIGURE 1.
FIGURE 3 shows a flowchart showing an exemplary method of use of the apparatus of FIGURE 1.

### DETAILED DESCRIPTION

Embodiments disclosed herein extend the wireless sensor communication range outside of the Faraday cage-shielded magnet room to neighboring areas (e.g. the MRI control room) while minimizing modifications to the patient monitor and maintaining integrity of the RF shielding of the magnet room. To this end, two radios can be provided - one inside the magnet room and one outside the magnet room (e.g. inside the adjacent control room) - which are connected by an optical fiber link passing through the RF shielding. The inside (or "first") radio picks up sensor transmissions and communicates them to the outside (or "second") radio via the fiber optic link, and the outside radio re-broadcasts the same transmissions at their original carrier frequencies but with a predefined time lag.

With this approach, only software modifications are needed at the patient monitor, e.g. adjusting the firmware to check the outside radio repeater time slots if the primary sensor signal becomes unreliable. The re-broadcasts are preferably at the same carrier frequency and use the same modulation and data encoding so no modification to the radio hardware is needed. The predefined time lag ensures that if both the broadcast and the re-broadcast are in range (e.g., as may be the case if the door between the control room and the magnet room is open) then the original sensor broadcast and the rebroadcast can be distinguished. Various approaches can be used to select the signal (broadcast or rebroadcast) for use at the patient monitor. In one approach, the broadcast is used unless its signal strength is too low (or the signal is poor by some other metric, e.g. fraction of bad packets) at which point the monitor switches to the rebroadcast. In another approach, the strongest signal is chosen.

To handle multiple data sources simultaneously, whether from wireless sensors or medical devices attached to physical ports, a multiplexor is provided with the inside radio to multiplex the sensor signals onto the optical fiber link. Each sensor usually broadcasts on a different channel (i.e. different carrier frequency), and the same solution for signal differentiation currently used by the patient monitor to capture these sensor signals at different frequencies can be replicated at the inside radio. One suitable approach includes leveraging the ECG signal having large redundancies (transmits 3000 points/sec, ECG trace can be reconstructed with 200 points/sec) and the use of two antennae to employ antenna diversity to compensate RF reflections in the magnet room. Another approach, albeit costlier in hardware, is to provide a set of receivers at the patient monitor for the different sensor channels. At the outside radio, the multiplexed signal is demultiplexed and a similar approach is used to rebroadcast the original sensor signals at the appropriate radio frequencies. It will also be appreciated that analogous hardware could be used to rebroadcast a signal originating in the control room in the magnet room.

In another embodiment, the outside radio is replaced by or augmented with one or more physical electrical data ports (Ethernet, serial, etc.) that mimic output physical electrical data ports of medical devices located inside the magnet room. The inside radio is similarly replaced by or augmented with mating electrical data ports designed to connect with the physical electrical data ports of the medical devices. In this way, the medical devices can be plugged into the mating ports located inside the magnet room, the signals from the ports are bundled together and transmitted over the non-electrical fiber optic link, and are de-bundled outside the magnet room and the constituent signals distributed to the appropriate physical electrical data ports mimicking the instrument electrical data ports. Thus, a user can "plug into" the medical device remotely, e.g. in the control room, by plugging into the output physical electrical data port of the outside radio corresponding to the physical port of the medical device located inside the magnet room. The addition of a Hospital Information System (HIS) port at the outside is also contemplated, so that signals from instruments inside the magnet room can be recorded in the HIS. Note that in this embodiment there is no need for the predefined time lag.

The use of a fiber optic link is advantageous for connecting the inside and outside radios due to its high speed and bandwidth (e.g. 5 megabits/sec readily achievable) which facilitates maintaining the design-basis time lag between the original sensor broadcast and the re-broadcast, although other RF shielding-compliant non-electrical links such as a laser/window/photodiode optical link are also contemplated.

One embodiment includes a digital repeater comprising an RF transceiver located in the magnet room, a fiber optic link through a waveguide to the control room, and an RF transceiver located in the control room. Data transmission in the control room is time synchronized so as to allow for a receiving device such as a monitor to seamlessly receive data in the magnet room, control room, or somewhere in between. In addition, a data port is provided in the control room radio to allow the received data to be sent to a HIS.

As used herein, the term "broadcast" (and variants thereof) refers to as a nondirectional wireless transmission (as opposed to a point-to-point directed wireless transmission, e.g. using a directional beam antenna or a laser beam) that can be received by any suitably tuned radio within a broadcast range of the transmitter. For example, the term "broadcast" can refer to a transmission of a signal from a physiological sensor to a first radio.

As used herein, the term "re-broadcast" (and variants thereof) refers to as a nondirectional wireless transmission that can be received by any suitably tuned radio within a predefined (re-)broadcast range. For example, the term "re-broadcast" can refer to a transmission of a signal from a second radio to a patient monitor.

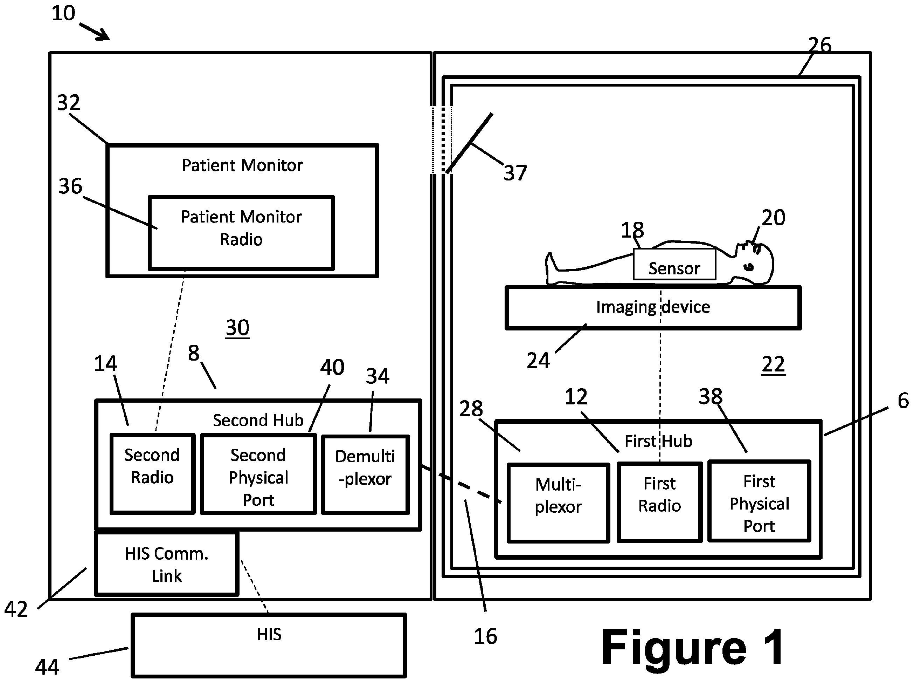

With reference to FIGURE 1, an embodiment of an apparatus **10** for processing physiological data streams is shown. As shown in FIGURE 1, the apparatus **10** includes a first hub **6** with a first radio **12,** a second hub **8** with a second radio **14,** and a non-electrical communication link **16** between the first and second hubs. Each of these components is described in more detail below.

The first radio **12** is configured to receive an individual medical information data stream broadcast by the at least one associated physiological sensor **18** at a data stream frequency and to transmit a non-electrical signal carrying the individual physiological data stream. For example, the medical information can include information about the patient, including physiological data obtained by the sensor **18,** information related to battery life, software version, drub library version, malfunction alerts, general alerts, high bandwidth data, and the like. A patient **20** can be positioned in a first room **22** with an imaging device **24** (e.g., a magnetic resonance (MR) imaging device, a positron emission tomography (PET) imaging device, a single-positron emission computed tomography imaging device, and the like). It should be noted that the imaging device **24** is diagrammatically indicated in FIGURE 1, and may for example be a horizontal bore-type MRI with the patient **20** loaded into the MRI bore (and hence substantially occluded from view), an open MRI, or so forth. The first room **22** includes a radio frequency (RF) shield **26** that surrounds the first room (e.g., by being embedded in walls of the room). Such a radio frequency shield is commonly used in conjunction with an MRI and, as such, the first room **22** can be referred to as a "magnet room" and the RF shield **26** forms a Faraday cage **26** enclosing the magnet room **22.** However, other types of imaging systems may benefit from Faraday cage shielding, e.g. a PET scanner may be sensitive to outside RF interference. The physiological sensors **18** (e.g., an SpO2 sensor, an ECG sensor, a temperature sensor, a blood pressure sensor, and the like) can be operably connected to the patient **20.** In the case of sensors **18** used to monitor a patient **20** loaded into the MRI examination region and hence exposed to strong magnetic fields, magnetic field gradients, and RF pulses, the sensor should be MRI compatible. Using a wireless sensor is one way to improve MRI compatibility as this eliminates electrically conductive wires that could otherwise couple with magnetic field gradients and heat up due to induced eddy currents. The sensor **18** for MRI compatibility is also preferably free of ferromagnetic material and may incorporate other features such as perforated RF shielding (if such shielding is needed) to suppress eddy currents.

The first radio **12** is configured to receive physiological data streams from each of the physiological sensors **18** and transmit the data streams to the second radio **14.** To do so, the first hub **6** includes a multiplexor or bundler **28.** The multiplexor **28** is programmed to receive a plurality of individual physiological data streams from the physiological sensors **18.** For example, the multiplexor **28** is programmed to receive an SpO2 signal from the SpO2 sensor, an ECG signal from the ECG sensor, and so on. The multiplexor **28** is then programmed to bundle each of these physiological data streams into a single data stream. For example, the multiplexor **28** bundles the individual data streams into a non-electrical signal. Once the single data stream is generated, the multiplexor **28** is then programmed to transmit the non-electrical signal carrying the single data stream via the non-electrical communication link **16** to the second radio **14.**

The second hub **8** is configured to receive the non-electrical signal carrying the physiological data stream from the first radio **12** and re-broadcast using the second radio **14** the physiological data stream at the data stream frequency with a defined time lag respective to the broadcast by the physiological sensors **18.** The second radio **14** is located in a second room **30** that is separate from the first room **22.** The second room **30** does not include an RF shield. As such, the second room 30 can be referred to as a "control room."

The second radio **14** is configured to receive the single, bundled data stream from the first radio **12,** and transmit the data stream to a patient monitor **32** (described in more detail below). To do so, the second hub **8** includes a de-multiplexer or de-bundler **34.** The de-multiplexor is programmed to receive the bundled, single data stream from the non-electrical communication link **16** via the multiplexor **28** of the first radio **12.** For example, the de-multiplexor **34** is programmed to de-bundle the single data stream into the individual data streams. For example, the de-multiplexor **34** de-bundles the single data stream back into the individual data streams originally transmitted by the physiological sensors **18** to the first radio **12.** Once the single data stream is de-bundled, the de-multiplexor **34** is then programmed to retransmit the individual data streams to the patient monitor **32,** as described in more detail below.

The non-electrical communication link **16** is configured to connect the first hub **6** and the second hub **8.** For example, the non-electrical communication link **16** is configured to be operably connected to the first radio **12** in the magnet room **22,** and the first second radio in the control room **30** by passing through the RF shield **26** (i.e., through a wall separating the magnet and control rooms **22** and **30**). As a result, the non-electrical signal carrying the individual physiological data stream is communicated from the first radio **12** to the second radio **14.** In some embodiments, the non-electrical communication link **16** can be configured as a fiber optic cable. Advantageously, the use of a fiber optic link is preferred due to its high speed and bandwidth (e.g. 5 megabits/sec readily achievable) which facilitates maintaining the design-basis time lag between an original sensor and a re-broadcast, as described in more detail below.

It should be noted that the inside and outside radios **12, 14** are shown diagrammatically in FIGURE 1. In a preferred implementation, each radio is a compact transceiver small enough to fit into a small space, e.g. the size of a cellular telephone (cellphone) in some embodiments, disposed in a corner of the respective magnet room **22** or control room **30** or mounted on a wall thereof, so as to be out-of-the-way of the radiological technician or other personnel. The two radios **12, 14** are preferably (although not necessarily) placed close to each other on opposite sides of the wall between the adjoining magnet room **22** and control room **30,** so that the optical fiber **16** can be of short length.

In some embodiments, the apparatus **10** can include a patient monitor **32.** The patient monitor **32** is configured to receive the individual physiological data streams broadcast by the physiological sensors **18** at the data stream frequency at which the sensors transmit the data streams to the first radio **12.** To do so, the patient monitor **32** includes a patient monitor radio **36** configured to receive the data streams from the sensors **18.** In addition, the patient monitor radio **36** is configured to receive a re-broadcast from the second radio **14** of the physiological data stream at the data stream carrier frequency with the defined time lag. The patient monitor **32** is typically positioned in the magnet room **22** so that medical professionals in the control room **30** can view the sensor data on the patient monitor. Advantageously, the patient monitor **32** can be configured to be mobile (e.g. battery-powered and mounted on a wheeled support rack or wheeled support console) so that it can be moved back and forth from the magnet room **22** and the control room **30** via a door **37** (again it is to be understood that the FIGURE 1 is diagrammatic, and the patient monitor **32** and door **37** are respectively sized to allow the patient monitor to be wheeled through the door), thereby ensuring that the data streams from the sensors 18 are continuously transmitted and shown on the patient monitor. In another example, the patient monitor **32** can be configured as a hand-held device, thereby allowing a technician to walk back and forth between the magnet room **22** and the control room **30** while carrying or holding the patient monitor.

Stated another way, the patient monitor **32** is configured to receive: (1) an original broadcast of the data streams from the sensors **18;** and (2) a re-broadcast of the same data streams from the second radio **14.** The patient monitor **32** is configured to select one of the broadcast and the re-broadcast and to read the physiological data stream from the selected one of the broadcast and the re-broadcast. In one example, the patient monitor **32** is configured to select one of the broadcast and the re-broadcast based on a signal strength metric of at least the broadcast. In one such approach, the broadcast is used unless its signal strength is too low (or the signal is poor by some other metric, e.g. fraction of bad packets) at which point the monitor switches to the rebroadcast. In another signal strength-based approach, the strongest signal is chosen. It will be appreciated that because of the Faradays shield **26,** the strongest signal is likely to be the direct broadcast from the sensor **18** when the patient monitor **32** is located inside the magnet room **22;** whereas, the strongest signal is likely to be the re-broadcast from the second radio **14** when the patient monitor **32** is located in the control room **30.** If the patient monitor **32** is located in the doorway of the door **37** then both the broadcast and the re-broadcast may be received with relatively strong signals, and one signal is selected preferably on the basis of signal strength. It will be noted that even if the door **37** is merely open this may be enough to reduce effectiveness of the Faraday cage **26** to an extent allowing both the broadcast and re-broadcast to be received at the patient monitor **32.**

Although signal strength is generally a preferred basis for selecting between the broadcast and re-broadcast, other criteria are contemplated to be used in the selection. For example, if the broadcast is initially selected and processed but a checksum data verification fails, then the re-broadcast may instead be processed. In some embodiments, as shown in FIGURE 1, the first hub **6** includes at least one first physical electrical data port **38** corresponding to one or more associated medical devices (e.g., an infusion pump, an anesthesiology machine, and the like). For example, the first physical electrical data ports **38** typically contain patient data for the associated medical device (e.g., data for an infusion pump that can include the type of drug, concentration, time start, alarm, etc.). Each first electrical data port **38** is configured to receive physiological data from a medical device in the magnet room **22** (not shown, e.g., an infusion pump, anesthesiology machine, or so forth). Similarly, the second hub **8** includes at least one second physical electrical data port **40** corresponding to each of the first ports **38,** and, as a result, are associated with the medical devices (such as the infusion pump, the anesthesiology machine, and the like). Each second physical electrical data port **40** is configured to receive the physiological data from the corresponding first physical electrical data port **38** via the non-electrical communication link **16.** Each second physical electrical data port **40** is designed to mimic the output port of a medical device for which it serves as a "remote surrogate" output. Thus, for example, the second physical electrical data port **40** has the same connector type and outputs data using the same format as the output port of the medical device. In this way, a user can plug into the second physical electrical data port **40** in the control room **30** and receive data identically to the way the user would plug into the output port of the medical device located inside the magnet room **22.**

In other embodiments, referring back to FIGURE 1, the apparatus **10** can also include a hospital information system communication link **42** configured to transmit data from the second hub **8** to an associated hospital information system **44.** For example, the data streams sent by the sensors **18** can be transmitted to the hospital information system communication link **42** so that the hospital information system **44** can be advantageously continuously updated with information related to the patient **20.** In other words, the data sent to the hospital information system **44** may include a patient identifier and the time. In another example, when a patient identification is not known, a machine ID for the sensors **18** or medical devices (not shown) can be used. The hospital information system **44** can resolve the patient identification since the hospital information system knows which patient was connected to which sensor **18** or medical device at a selected time. The hospital information system communication link **42** can be configured as a serial port. In some embodiments, the hospital information system communication link **42** is disposed on a portion of the second radio **14;** however, it will be appreciated that the hospital information system communication link **42** can be disposed in any suitable location (e.g., on the patient monitor **32,** separately from the components of the apparatus **10,** and the like).

It will also be appreciated that the various signals and values describe herein can be communicated to the various components **12, 14, 18, 32** and data processing components **28, 34** via a communication network (e.g., a wireless network, a local area network, a wide area network, a personal area network, BLUFTOOTH®, and the like). In another contemplated embodiment, the output of the sensors **18** is suitably displayed on the patient monitor **32.**

### EXAMPLE

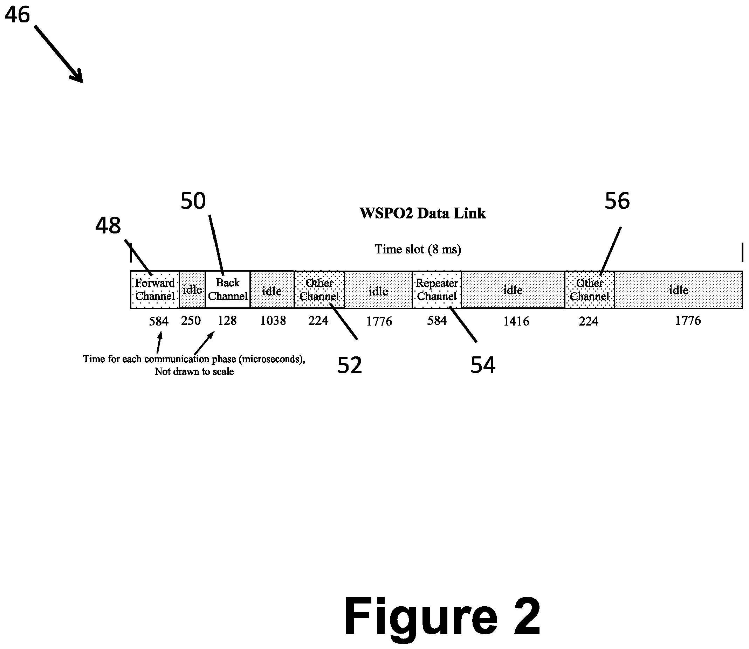

FIGURE 2 shows an example of a time line **46** during the operation of the apparatus **10.** In this example, the only physiological sensor **18** connected to the patient **20** is an SpO2 sensor. The sensor **18** is configured to transmit a plurality of data packets in a forward channel at 125 Hz. At this frequency, one packet is transmitted every 8 milliseconds (i.e., 8000 microseconds, which is the length of the time line **46**) on a defined carrier frequency FS_{PO2}. (It is to be understood that the carrier frequency is different from the packet frequency. For example, if a 2.4 GHz radio is being used then the carrier frequency is around 2.4 GHz; whereas, the packet frequency in this example is 125 Hz.) The sensor **18** is configured to transmit the data packets to the first radio **12** in the forward channel during a forward channel time period **48,** which lasts approximately 584 microseconds, and contains a rolling window of three successive samples. After an idle time of approximately 250 microseconds, the sensor **18** receives data in a back channel during a back channel time period **50,** which lasts approximately 128 microseconds long.. The speed of the fiber data transmission is chosen to be at a sufficient rate to allow time synchronization between packet protocol at the first radio **12** and packet protocol at the second radio **14;** such as 5 Mbps high speed serial or 100 Mbps Ethernet packet protocol.

The (illustrative) SpO2 data packets are received by the first radio **12** in the magnet room **22,** and transmitted to the second radio **14** in the control room **30** via the fiber optic cable **16.** However, the carrier frequency F_{SPO2} may contain traffic from other devices (e.g., other sensors **18,** the patient monitor **32,** the imaging device **24,** and the like) located in the magnet room **22.** When the door **37** between the magnet room **22** and the control room **30** is open, the sensors **18,** the second radio **14,** and the patient monitor radio **36** may all be in range of each other. Therefore, it is important that the second radio **14** located in the control room **30** be configured transmit at a specific time so as not to interfere with the other packet traffic. Time synchronization between the two radios can be achieved using an Ethernet protocol, application level packets, or other packet synchronization protocol.

After another idle time period of approximately 1.038 milliseconds, the data stream is transmitted from other equipment (e.g., the other sensors **18,** the patiet monitor **32,** an infusion pump, a ventilator, an anesthesia machine, and the like) in the magnet room **22.** This transmission occurs during a first other channel time period **52,** which lasts approximately 224 microseconds. In addition, after another idle time period of approximately 1.776 milliseconds, the second radio **14** can send the data packet directly to the patient monitor radio **36** during a repeater time period **54** which lasts approximately 584 microseconds. In this manner, the second radio **14** is receiving the same data stream from different sources (i.e., the first radio **12** and the sensor **18**) at different times to avoid other traffic.

After yet another idle time of approximately 1.416 milliseconds, the other equipment is configured to send the data packets to the patient monitor **32** during a second other channel time period **56,** which lasts approximately 224 microseconds. Again, the idle period allows the second radio **14** to transmit the data to the patient monitor **32** to avoid other data transmission traffic. In some examples, when the patient monitor **32** is located in the control room **30,** back channel packets can be sent to the magnet room **22** or retransmission to the sensor **18** at the appropriate time. Finally, after another idle period that lasts approximately 1.776 milliseconds, the process repeats with the sensor **18** transmitting another data packet to the first radio **12** in the forward channel during a forward channel time period **48.**

In this manner, in the magnet room **22,** the patient monitor **32** can directly receive original sensor data from the sensor **18.** When the patient monitor **32** is moved to another location, such as the doorway between the magnet room **22** and the control room, the patient monitor **32** can receive both the original and repeated forward channel packets. In the control room **30** with the door **37** closed, the patient monitor **32** can receive repeated packets only. This allows the patient monitor **32** to be moved back and forth between the rooms **22, 30** and continuously display patient information.

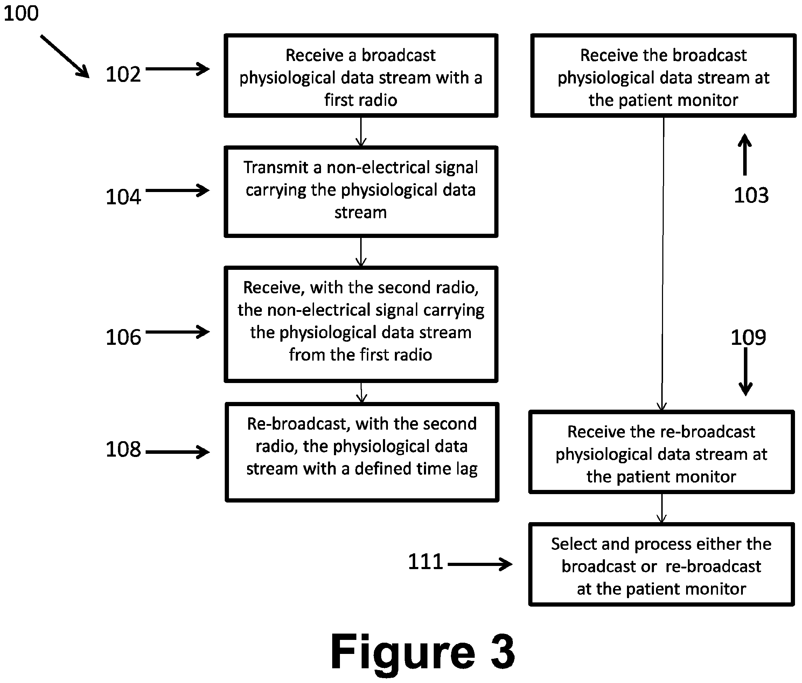

FIGURE 3 shows a method **100** of for processing physiological data streams broadcast by at least one associated physiological sensor **18.** The method **100** includes receiving, with a first hub **6** including a first radio **12,** an individual physiological data stream broadcast by the at least one associated physiological sensor **18** at a data stream carrier frequency **(102).** At the same time, the broadcast is also received by the patient monitor **32 (103),** if the patient monitor is in range (e.g. inside the magnet room **22**). With the first radio **12,** a non-electrical signal carrying the individual physiological data stream is transmitted to a second hub **8** including a second radio **14** via a non-electrical communication link **16** between the first radio **12** and the second radio **14 (104).** At the second radio **14,** the non-electrical signal carrying the physiological data stream is received from the first radio **12 (106).** The second radio **14** re-broadcasts the physiological data stream at the data stream carrier frequency with a defined time lag respective to the broadcast by the at least one associated physiological sensor **18 (108).** The re-broadcast is received by the patient monitor **32 (109),** if the patient monitor is in range (e.g. inside the control room **22**). The patient monitor then selects and processes (e.g. displays the data trend from) either the broadcast or the re-broadcast **(111),** depending upon which has the best signal strength or depending on some other selection criterion.

The various components **6, 8, 12, 14, 18, 32** of the apparatus **10** can include at least one microprocessor **28, 34** programmed by firmware or software to perform the disclosed operations. In some embodiments, the microprocessor **28, 34** is integral to the various component **12, 14, 18, 32,** so that the data processing is directly performed by the various component **12, 14, 18, 32.** In other embodiments the microprocessor **28, 34** is separate from the various component **12, 14, 18, 32.** The data processing components **28, 34** of the apparatus **10** may also be implemented as a non-transitory storage medium storing instructions readable and executable by a microprocessor (e.g. as described above) to implement the disclosed operations. The non-transitory storage medium may, for example, comprise a read-only memory (ROM), programmable read-only memory (PROM), flash memory, or other repository of firmware for the various components **12, 14, 18, 32** and data processing components **28, 34.** Additionally or alternatively, the non-transitory storage medium may comprise a computer hard drive (suitable for computer-implemented embodiments), an optical disk (e.g. for installation on such a computer), a network server data storage (e.g. RAID array) from which the various component **6, 8, 12, 14, 18, 32,** data processing components **28, 34,** or a computer can download the apparatus software or firmware via the Internet or another electronic data network, or so forth.

The invention has been described with reference to the preferred embodiments. Modifications and alterations may occur to others upon reading and understanding the preceding detailed description. 2. IF The invention is defined by appended claims 1-15.

## Claims

1. An apparatus **(10)** for processing medical information data streams broadcast by at least one associated physiological sensor **(18),** the apparatus comprising:
a first hub **(6)** including a first radio **(12)** configured to receive an individual medical information data stream broadcast by the at least one associated sensor (18) at a data stream carrier frequency and to transmit a non-electrical signal carrying the individual medical information data stream;
a second hub **(8)** including a second radio **(14);** and
a non-electrical communication link **(16)** between the first hub **(6)** and the second hub **(8)** via which the non-electrical signal carrying the individual medical information data stream is communicated from the first radio **(12)** to the second radio **(14);**
the second radio **(14)** being configured to receive the non-electrical signal carrying the medical information data stream from the first radio **(12)** and re-broadcast the medical information data stream at the data stream carrier frequency with a defined time lag respective to the broadcast by the at least one associated sensor **(18)** such that the individual medical information data stream broadcast by the at least one sensor **(18)** and the re-broadcast can be distinguished;
wherein the non-electrical communication link **(16)** is a fiber optic cable **(16).**

2. The apparatus **(10)** of claim 1, further comprising:
a patient monitor **(32)** including a radio **(36)** configured to:
receive the individual medical information data stream broadcast by the at least one sensor **(18)** at the data stream carrier frequency; and
receive a re-broadcast from the second radio **(14)** of the medical information data stream at the data stream carrier frequency with the defined time lag.

3. The apparatus **(10)** of claim 2, wherein:
the first hub **(6)** includes a multiplexer **(28)** programmed to:
receive a plurality of individual medical information data streams from one or more sensors **(18);**
bundle the plurality of individual medical information data streams into a single data stream; and
transmit the non-electrical signal carrying the single data stream via the non-electrical communication link **(16)** to the second radio **(14);** and
the second hub **(8)** includes a de-multiplexer **(34)** programmed to:
receive the single data stream from the non-electrical communication link **(16);**
de-bundle the single data stream into the individual data streams; and
retransmit the individual data streams to the patient monitor **(32).**

4. The apparatus **(10)** of claim 2, wherein the patient monitor **(32)** is further configured to select one of the broadcast and the re-broadcast and to read the medical information data stream from the selected one of the broadcast and the re-broadcast.

5. The apparatus **(10)** of claim 4, wherein the patient monitor **(32)** is configured to select one of the broadcast and the re-broadcast based on a signal strength metric of at least the broadcast.

6. The apparatus **(10)** of any one of claims 1-5, wherein
the first hub **(6)** includes at least one first physical port **(38)** corresponding to each of the associated sensors **(18),** each first port **(38)** being configured to receive medical information data from the corresponding sensor; and
the second hub **(8)** includes at least one second physical port **(40)** corresponding to each of the first ports **(38),** each second port **(40)** being configured to receive the medical information data from the corresponding first port **(38)** via the non-electrical communication link **(16).**

7. The apparatus **(10)** of any one of claims 1-6, wherein the second hub **(8)** includes a hospital information system communication link **(42)** configured to transmit data from the second radio **(14)** to an associated hospital information system **(44).**

8. A magnetic resonance imaging (MRI) system comprising:
an MRI imaging device **(24)** disposed in a magnet room **(22);**
a Faraday cage **(26)** enclosing the magnet room; and
the apparatus **(10)** of any one of claims -7 for processing medical information data streams broadcast by at least one associated sensor **(18)** disposed on a patient in the MRI imaging device, wherein the first hub **(6)** is disposed inside the Faraday cage, the second hub **(8)** is disposed outside the Faraday cage, and the non-electrical communication link **(16)** comprises a fiber optic cable passing through the Faraday cage.

9. A method **(100)** for processing physiological data streams broadcast by at least one associated physiological sensor **(18),** the method comprising:
receiving, with a first hub **(6)** including a first radio **(12),** an individual physiological data stream broadcast by the at least one associated physiological sensor **(18)** at a data stream carrier frequency;
transmitting, with a first radio **(12),** a non-electrical signal carrying the individual physiological data stream to a second hub **(8)** including a second radio **(14)** via a non-electrical communication link **(16)** between the first radio **(12)** and the second radio **(14);**
receiving, with the second radio **(14),** the non-electrical signal carrying the physiological data stream from the first radio **(12);** and
re-broadcasting, with the second radio **(14),** the physiological data stream at the data stream carrier frequency with a defined time lag respective to the broadcast by the at least one associated physiological sensor **(18)** such that the individual medical information data stream broadcast by the at least one sensor **(18)** and the re-broadcast can be distinguished,
wherein the non-electrical signal is transmitted via a fiber optic cable **(16)** connecting the first hub **(6)** and the second hub **(8).**

10. The method **(100)** of claim 9, further comprising:
with a patient monitor **(32)** including a radio **(36):**
receiving the individual physiological data stream broadcast by the at least one physiological sensor **(18)** at the data stream carrier frequency; and
receiving a re-broadcast from the second radio **(14)** of the physiological data stream at the data stream carrier frequency with the defined time lag.

11. The method **(100)** of claim 10, further including:
with the first hub **(6),**
receiving a plurality of individual physiological data streams from the at least one associated physiological sensor **(18);**
bundling each physiological data stream into a single data stream; and transmitting the non-electrical signal carrying the single data stream via the non-electrical communication link **(16)** to the second radio **(14);** and
with the second hub **(8),**
receiving the single data stream from the non-electrical communication link **(16);**
de-bundling the single data stream into the individual data streams; and retransmitting the individual data streams to the patient monitor **(32).**

12. The method **(100)** of claim 11, further including:
with the patient monitor **(32),** selecting one of the broadcast and the re-broadcast and to read the physiological data stream from the selected one of the broadcast and the re-broadcast.

13. The method **(100)** of claim 12, further including:
with the patient monitor **(32),** selecting one of the broadcast and the re-broadcast based on a signal strength metric of at least the broadcast.

14. The method **(100)** of any one of claims 9-13, wherein
with at least one first physical port **(38)** of the first hub **(6)** corresponding to each of the associated physiological sensors **(18),** receiving physiological data from the corresponding physiological sensor **(18);** and
with at least one second physical port **(40)** of the second hub **(8)** corresponding to each of the first ports **(38),** receiving the physiological data from the corresponding first port **(38)** via the non-electrical communication link **(16).**

15. The method **(100)** of any one of claims 9-14, further including:
with a hospital information system communication link **(42)** of the second hub **(8),** transmitting data from the second radio **(14)** to an associated hospital information system **(44).**

## Patentansprüche

1. Gerät **(10)** zum Verarbeiten von medizinischen Informationsdatenströmen, die von mindestens einem zugeordneten physiologischen Sensor **(18)** gesendet werden, wobei das Gerät Folgendes umfasst:
einen ersten Hub **(6),** der ein erstes Funkgerät **(12)** umfasst, das einen einzelnen medizinischen Informationsdatenstrom empfängt, der vom mindestens einen zugehörigen Sensor **(18)** auf einer Datenstromträgerfrequenz gesendet wird, und dass ein nicht-elektrisches Signal sendet, das den einzelnen medizinischen Informationsdatenstrom enthält;
einen zweiten Hub **(8),** der ein zweites Funkgerät **(14)** umfasst; und
eine nicht-elektrische Kommunikationsverbindung **(16)** zwischen dem ersten Hub **(6)** und dem zweiten Hub **(8),** über die das nicht-elektrische Signal, das den einzelnen medizinischen Informationsdatenstrom enthält, vom ersten Funkgerät **(12)** an das zweite Funkgerät **(14)** übertragen wird;
wobei das zweite Funkgerät **(14)** das nicht-elektrische Signal, das den medizinischen Informationsdatenstrom enthält, vom ersten Funkgerät **(12)** empfängt und den medizinischen Informationsdatenstrom auf der Datenstromträgerfrequenz mit einer definierten Zeitverzögerung für das Senden durch den mindestens einen zugehörigen Sensor **(18)** erneut sendet, sodass der einzelne, vom mindestens einen Sensor **(18)** gesendete medizinische Informationsdatenstrom vom erneuten Senden unterschieden werden kann;
wobei es sich bei der nicht-elektrischen Kommunikationsverbindung **(16)** um einen Lichtwellenleiter **(16)** handelt.

2. Das Gerät **(10)** gemäß Anspruch 1, das zudem Folgendes umfasst:
ein Patientenüberwachungsgerät **(32),** das ein Funkgerät **(36)** umfasst, das folgende Schritte durchführt:
Empfangen des einzelnen medizinischen Informationsdatenstroms, der vom mindestens einen Sensor **(18)** auf der Datenstromträgerfrequenz gesendet wird; und
Empfangen des mit der definierten Zeitverzögerung auf der Datenstromträgerfrequenz erneut gesendeten medizinischen Informationsdatenstroms vom zweiten Funkgerät **(14).**

3. Das Gerät **(10)** gemäß Anspruch 2, wobei:
der erste Hub **(6)** einen Multiplexer **(28)** umfasst, der folgende Schritte durchführt:
Abrufen mehrerer einzelner medizinischer Informationsdatenströme von mindestens einem Sensor **(18);**
Bündeln der einzelnen medizinischen Informationsdatenströme in einem einzigen Datenstrom; und
Übertragen des nicht-elektrischen Signals, das den einzelnen Datenstrom enthält, über die nicht-elektrische Kommunikationsverbindung **(16)** an das zweite Funkgerät **(14);** und
der zweite Hub **(8)** einen Demultiplexer **(34)** umfasst, der folgende Schritte durchführt:
Empfangen des einzelnen Datenstroms über die nicht-elektrische Kommunikationsverbindung **(16);**
Entbündeln des einzelnen Datenstroms in die einzelnen Datenströme; und
erneutes Übertragen der einzelnen Datenströme an das Patientenüberwachungsgerät **(32).**

4. Das Gerät **(10)** gemäß Anspruch 2, wobei das Patientenüberwachungsgerät **(32)** zudem entweder das erste oder das erneute Senden auswählt und den medizinischen Informationsdatenstrom aus dem ausgewählten ersten oder erneuten Senden ausliest.

5. Das Gerät **(10)** gemäß Anspruch 4, wobei das Patientenüberwachungsgerät **(32)** entweder das erste oder das erneute Senden anhand einer Signalstärke-Metrik mindestens des ersten Sendens auswählt.

6. Das Gerät **(10)** gemäß einem der Ansprüche 1 bis 5, wobei
der erste Hub **(6)** mindestens einen ersten physikalischen Anschluss **(38)** umfasst, der jeweils einem der zugehörigen Sensoren **(18)** entspricht, wobei die einzelnen ersten Anschlüsse **(38)** medizinische Informationsdaten vom entsprechenden Sensor empfangen; und der zweite Hub **(8)** mindestens einen zweiten physikalischen Anschluss **(40)** umfasst, der jeweils einem der ersten Anschlüssen **(38)** entspricht, wobei die einzelnen zweiten Anschlüsse **(40)** über die nicht-elektrische Kommunikationsverbindung **(16)** medizinische Informationsdaten vom entsprechenden ersten Anschluss **(38)** empfangen.

7. Das Gerät **(10)** gemäß einem der Ansprüche 1 bis 6, wobei der zweite Hub **(8)** eine Kommunikationsverbindung des Krankenhausinformationssystems **(42)** umfasst, die Daten vom zweiten Funkgerät **(14)** an das zugehörige Krankenhausinformationssystem **(44)** überträgt.

8. Ein Magnetresonanztomographie-System (MRT), das Folgendes umfasst:
ein MRT-Bildgebungsgerät **(24),** das sich in einem Magnetraum **(22)** befindet;
einen Faradayschen Käfig **(26),** der den Magnetraum umschließt; und
das Gerät **(10)** gemäß einem der Ansprüche 1 bis 7 zum Verarbeiten von medizinischen Informationsdatenströmen, die von mindestens einem zugehörigen, am Patienten befindlichen Sensor **(18)** im MRT-Bildgebungsgerät gesendet werden, wobei sich der erste Hub **(6)** im Faradayschen Käfig und der zweite Hub **(8)** außerhalb des Faradayschen Käfigs befindet, und wobei die nicht-elektrische Kommunikationsverbindung **(16)** ein durch den Faradayschen Käfig verlaufendes Lichtwellenleiterkabel umfasst.

9. Eine Methode **(100)** zum Verarbeiten von physiologischen Informationsdatenströmen, die von mindestens einem zugeordneten physiologischen Sensor **(18)** gesendet werden, wobei die Methode folgende Schritte umfasst:
Empfangen mit einem ersten Hub **(6),** der ein erstes Funkgerät **(12)** umfasst, eines individuellen physiologischen Datenstroms, der vom mindestens einen zugeordneten physiologischen Sensor **(18)** mit einer Datenstromträgerfrequenz gesendet wird;
Übertragen, mit einem ersten Funkgerät **(12),** eines nicht-elektrischen Signals, das den einzelnen physiologischen Datenstrom enthält, über eine nicht-elektrische Kommunikationsverbindung **(16)** zwischen dem ersten Funkgerät **(12)** und dem zweiten Funkgerät **(14)** an den zweiten Hub **(8),** der ein zweites Funkgerät **(14)** umfasst;
Empfangen, mit dem zweiten Funkgerät **(14),** des nicht-elektrischen Signals vom ersten Funkgerät **(12),** das den physiologischen Datenstrom enthält; und
erneutes Senden, mit dem zweiten Funkgerät **(14),** des physiologischen Informationsdatenstroms auf der Datenstromträgerfrequenz mit einer definierten Zeitverzögerung für das Senden durch den mindestens einen zugehörigen physiologischen Sensor **(18),** sodass der einzelne, vom mindestens einen Sensor **(18)** gesendete medizinische Informationsdatenstrom vom erneuten Senden unterschieden werden kann,
wobei das nicht-elektrische Signal über ein Lichtwellenleiter-Kabel **(16)** übertragen wird, das den ersten Hub **(6)** und den zweiten Hub **(8)** verbindet.

10. Die Methode **(100)** gemäß Anspruch 9, die zudem folgende Schritte umfasst:
mit einem Patientenüberwachungsgerät **(32),** das ein Funkgerät **(36)** umfasst:
Empfangen des einzelnen physiologischen Informationsdatenstroms, der vom mindestens einen physiologischen Sensor **(18)** auf der Datenstromträgerfrequenz gesendet wird; und
Empfangen des mit der definierten Zeitverzögerung auf der Datenstromträgerfrequenz erneut gesendeten physiologischen Informationsdatenstroms vom zweiten Funkgerät **(14).**

11. Die Methode **(100)** gemäß Anspruch 10, die zudem folgende Schritte umfasst:
mit dem ersten Hub **(6):**
Empfangen mehrerer einzelner physiologischer Informationsdatenströme von mindestens einem zugehörigen physiologischen Sensor **(18);**
Bündeln der einzelnen physiologischen Datenströme in einen einzelnen Datenstrom; und
Übertragen des nicht-elektrischen Signals, das den einzelnen Datenstrom enthält, über die nicht-elektrische Kommunikationsverbindung **(16)** an das zweite Funkgerät **(14);** und
mit dem zweiten Hub **(8):**
Empfangen des einzelnen Datenstroms über die nicht-elektrische Kommunikationsverbindung **(16);**
Entbündeln des einzelnen Datenstroms in die einzelnen Datenströme; und
erneutes Übertragen der einzelnen Datenströme an das Patientenüberwachungsgerät **(32).**

12. Die Methode **(100)** gemäß Anspruch 11, die zudem folgende Schritte umfasst:
mit dem Patientenüberwachungsgerät **(32)** Auswählen entweder des ersten oder des erneuten Sendens Auslesen des medizinischen Informationsdatenstroms aus dem ausgewählten ersten oder erneuten Senden.

13. Die Methode **(100)** gemäß Anspruch 12, die zudem folgende Schritte umfasst:
Mit dem Patientenüberwachungsgerät **(32)** Auswählen entweder des ersten oder des erneuten Sendens anhand einer Signalstärke-Metrik mindestens des ersten Sendens.

14. Die Methode **(100)** gemäß einem der Ansprüche 9 bis 13, wobei
über mindestens einen ersten physikalischen Anschluss **(38)** des ersten Hubs **(6),** der jeweils einem der zugehörigen physiologischen Sensoren **(18)** entspricht, physiologische Daten vom entsprechenden physiologischen Sensor **(18)** empfangen werden; und
über mindestens einen zweiten physikalischen Anschluss **(40)** des zweiten Hubs **(8),** der jeweils einem der ersten Anschlüsse **(38)** entspricht, über die nicht-elektrische Kommunikationsverbindung **(16)** physiologische Daten vom entsprechenden ersten Anschluss **(38)** empfangen werden.

15. Die Methode **(100)** gemäß einem der Ansprüche 9 bis 14, die zudem folgenden Schritt umfasst:
über eine Kommunikationsverbindung des Krankenhausinformationssystems **(42)** des zweiten Hubs **(8)** Übertragen von Daten vom zweiten Funkgerät **(14)** an ein zugehöriges Krankenhausinformationssystem **(44).**

## Revendications

1. Appareil **(10)** de traitement des flux de données d'informations médicales diffusés par au moins un capteur physiologique **(18)** associé, ledit appareil comprenant :
un premier concentrateur **(6)** comprenant une première radio **(12)** conçue pour recevoir un flux de données d'informations médicales individuel diffusé par l'au moins un capteur **(18)** associé à une fréquence porteuse du flux de données et pour transmettre un signal non électrique transportant le flux de données d'informations médicales individuel ;
un deuxième concentrateur **(8)** comprenant une seconde radio **(14)** ; et
une liaison de communication non électrique **(16)** entre le premier concentrateur **(6)** et le second concentrateur **(8)** par l'intermédiaire de laquelle le signal non électrique transportant le flux de données d'informations médicales individuel est communiqué de la première radio **(12)** à la seconde radio **(14)** ;
la seconde radio **(14)** étant conçue pour recevoir le signal non électrique transportant le flux de données d'informations médicales de la première radio **(12)** et
pour rediffuser le flux de données d'informations médicales à la fréquence porteuse du flux de données à un décalage temporel défini respectif à la diffusion par l'au moins un capteur **(18)** associé de telle sorte que le flux de données d'informations médicales individuel diffusé par l'au moins un capteur **(18)** et la rediffusion puissent être distingués ;
dans lequel la liaison de communication non électrique **(16)** est un câble à fibre optique **(16).**

2. Appareil **(10)** selon la revendication 1, comprenant en outre :
un moniteur patient **(32)** comprenant une radio **(36)** conçue :
pour recevoir le flux de données d'informations médicales individuel diffusé par l'au moins un capteur **(18)** à la fréquence porteuse du flux de données ; et pour recevoir une rediffusion de la seconde radio **(14)** du flux de données d'informations médicales à la fréquence porteuse du flux de données au décalage temporel défini.

3. Appareil **(10)** selon la revendication 2, dans lequel :
le premier concentrateur **(6)** comprend un multiplexeur **(28)** programmé :
pour recevoir une pluralité de flux de données d'informations médicales individuels provenant d'au moins un capteur **(18)** ;
pour regrouper la pluralité de flux de données d'informations médicales individuels en un flux de données unique ; et
pour transmettre le signal non électrique transportant le flux de données unique par l'intermédiaire de la liaison de communication non électrique **(16)** à la seconde radio **(14)** ; et
le second concentrateur **(8)** comprend un démultiplexeur **(34)** programmé :
pour recevoir le flux de données unique de la liaison de communication non électrique **(16)** ;
pour dégrouper le flux de données unique en flux de données individuels ; et pour retransmettre les flux de données individuels au moniteur patient **(32).**

4. Appareil **(10)** selon la revendication 2, dans lequel le moniteur patient **(32)** est en outre conçu pour sélectionner la diffusion ou la rediffusion et pour lire le flux de données d'informations médicales à partir de la diffusion ou de la rediffusion sélectionnée.

5. Appareil **(10)** selon la revendication 4, dans lequel le moniteur patient **(32)** est conçu pour sélectionner la diffusion ou la rediffusion en fonction d'une métrique d'intensité du signal d'au moins la diffusion.

6. Appareil **(10)** selon l'une quelconque des revendications 1 à 5, dans lequel
le premier concentrateur **(6)** comprend au moins un premier port **(38)** physique correspondant à chacun des capteurs **(18)** associés, chaque premier port **(38)** étant conçu pour recevoir des données d'informations médicales du capteur correspondant ; et
le second concentrateur **(8)** comprend au moins un second port **(40)** physique correspondant à chacun des premiers ports **(38),** chaque second port **(40)** étant conçu pour recevoir les données d'informations médicales du premier port **(38)** correspondant par l'intermédiaire de la liaison de communication non électrique **(16).**

7. Appareil **(10)** selon l'une quelconque des revendications 1 à 6, dans lequel le second concentrateur **(8)** comprend une liaison de communication du système d'information hospitalière **(42)** conçue pour transmettre des données de la seconde radio **(14)** à un système d'information hospitalier **(44)** associé.

8. Système d'imagerie par résonance magnétique (IRM) comprenant :
un dispositif d'imagerie IRM **(24)** disposé dans une chambre magnétique **(22)** ;
une cage de Faraday **(26)** enfermant la chambre magnétique ; et
l'appareil **(10)** selon l'une quelconque des revendications 1 à 7, destiné au traitement des flux de données d'informations médicales diffusés par au moins un capteur **(18)** associé disposé sur un patient dans le dispositif d'imagerie IRM, dans lequel le premier concentrateur **(6)** est disposé à l'intérieur de la cage de Faraday, le second concentrateur **(8)** est disposé à l'extérieur de la cage de Faraday, et la liaison de communication non électrique **(16)** comprend un câble à fibre optique traversant la cage de Faraday.

9. Procédé **(100)** de traitement des flux de données physiologiques diffusés par au moins un capteur physiologique **(18)** associé, ledit procédé comprenant :
la réception, par un premier concentrateur **(6)** comprenant une première radio **(12),** d'un flux de données physiologiques individuel diffusé par l'au moins un capteur physiologique **(18)** associé à une fréquence porteuse du flux de données ;
la transmission, par une première radio **(12),** d'un signal non électrique transportant le flux de données physiologiques individuel vers un second concentrateur **(8)** comprenant une seconde radio **(14)** par l'intermédiaire d'une liaison de communication non électrique **(16)** entre la première radio **(12)** et la seconde radio **(14)** ;
la réception, par la seconde radio **(14),** du signal non électrique transportant le flux de données physiologiques de la première radio **(12)** ; et
la rediffusion, par la seconde radio **(14),** du flux de données physiologiques à la fréquence porteuse du flux de données à un décalage temporel défini respectif à la diffusion par l'au moins un capteur physiologique **(18)** associé de telle sorte que le flux de données d'informations médicales individuel diffusé par l'au moins un capteur **(18)** et la rediffusion peuvent être distinguées,
dans lequel le signal non électrique est transmis par l'intermédiaire d'un câble à fibre optique **(16)** reliant le premier concentrateur **(6)** et le second concentrateur **(8).**

10. Procédé **(100)** selon la revendication 9, comprenant en outre :
par un moniteur patient **(32)** comprenant une radio **(36)** ,
la réception du flux de données physiologiques individuel diffusé par l'au moins un capteur physiologique **(18)** à la fréquence porteuse du flux de données ; et
la réception d'une rediffusion de la seconde radio **(14)** du flux de données physiologiques à la fréquence porteuse du flux de données au décalage temporel défini.

11. Procédé **(100)** selon la revendication 10, comprenant en outre :
par le premier concentrateur **(6),**
la réception d'une pluralité de flux de données physiologiques individuels de l'au moins un capteur physiologique **(18)** associé ;
le regroupement de chaque flux de données physiologiques en un flux de données unique ; et
la transmission du signal non électrique transportant le flux de données unique par l'intermédiaire de la liaison de communication non électrique **(16)** à la seconde radio **(14)** ; et
par le second concentrateur **(8),**
la réception du flux de données unique de la liaison de communication non électrique **(16)** ;
le dégroupement du flux de données unique en flux de données individuels ; et la retransmission des flux de données individuels au moniteur patient **(32).**

12. Procédé **(100)** selon la revendication 11, comprenant en outre :
par le moniteur patient **(32),** la sélection de la diffusion ou de la rediffusion et la lecture du flux de données physiologiques à partir de la diffusion ou de la rediffusion sélectionnée.

13. Procédé **(100)** selon la revendication 12, comprenant en outre :
par le moniteur patient **(32),** la sélection de la diffusion ou de la rediffusion en fonction d'une métrique d'intensité du signal d'au moins la diffusion.

14. Procédé **(100)** selon l'une quelconque des revendications 9 à 13, comprenant en outre :
par au moins un premier port **(38)** physique du premier concentrateur **(6)** correspondant à chacun des capteurs physiologiques **(18)** associés, la réception des données physiologiques du capteur physiologique **(18)** correspondant ; et
par au moins un second port **(40)** physique du second concentrateur **(8)** correspondant à chacun des premiers ports **(38),** la réception des données physiologiques du premier port **(38)** correspondant par l'intermédiaire de la liaison de communication non électrique **(16).**

15. Procédé **(100)** selon l'une quelconque des revendications 9 à 14, comprenant en outre :
par une liaison de communication de système d'information hospitalière **(42)** du second concentrateur **(8),** la transmission des données de la seconde radio **(14)** à un système d'information hospitalier **(44)** associé.
